# EUROPÄISCHE PATENTSCHRIFT

(11) **EP 1 044 357 B1**
(45) Veröffentlichungstag und Bekanntmachung des Hinweises auf die Patenterteilung: **24.07.2002**
(21) Anmeldenummer: 98965176.5
(22) Anmeldetag: 25.11.1998
(51) Int. Cl.: G01N 1/34

(54) **VERFAHREN UND VORRICHTUNG ZUR QUASI-KONTINUIERLICHEN BESTIMMUNG VON ANORGANISCHEN ODER ORGANISCHEN INHALTSSTOFFEN IN FLUIDEN**
METHOD AND DEVICE FOR THE QUASI-CONTINUOUS ANALYSIS OF INORGANIC OR ORGANIC SUBSTANCES CONTAINED IN FLUIDS
PROCEDE ET DISPOSITIF DE MISE EN EVIDENCE QUASI-CONTINUE DE LA PRESENCE DE CONSTITUANTS INORGANIQUES OU ORGANIQUES DANS DES FLUIDES

(30) Priorität: 04.12.1997 DE 19753701
(43) Veröffentlichungstag der Anmeldung: 18.10.2000
(73) Patentinhaber: Fraunhofer-Gesellschaft zur Förderung der angewandten Forschung e.V., 80636 München (DE)
(72) Erfinder: GROTE, Christoph, D-37671 Höxter (DE); LEVSEN, Karsten, D-67112 Mutterstadt (DE)
(74) Vertreter: Pfenning, Meinig & Partner
(86) Internationale Anmeldenummer: EP9807594
(87) Internationale Veröffentlichungsnummer: WO9930128

(56) Entgegenhaltungen:
- WO-A-91/15745
- WO-A-97/25606
- US-A- 5 496 741
- EISERT R ET AL: "Development of a prototype system for quasi-continuous analysis of organic contaminants in surface or sewage water based on in-line coupling of solid-phase microextraction to gas chromatography" JOURNAL OF CHROMATOGRAPHY A, Bd. 737, Nr. 1, 14. Juni 1996, Seite 59-65 XP004039103

## Beschreibung

Die Erfindung betrifft ein Verfahren und eine Vorrichtung zur quasi-kontinuierlichen Bestimmung von anorganischen oder organischen Inhaltsstoffen in verschiedenen Fluiden, wobei günstigerweise die Überwachung organischer Kontaminationen in Trink-, Oberflächen- und Abwasser, wie letzteres beispielsweise in Industriebetrieben anfällt, durchgeführt werden kann. Eine solche Überwachung von Abwasserinhaltsstoffen kann vorteilhaft vor dem Einlauf in Kläranlagen durchgeführt werden, um insbesondere die Leistungsfähigkeit biologischer Stufen von Kläranlagen zu gewährleisten und durch erhöhte Konzentrationen hervorrufbare Störungen durch rechtzeitiges Erkennen zu verhindern. Eine Kontrolle des Kläranlagenablaufes ist ebenfalls möglich und sinnvoll, um den Eintrag toxischer Stoffe in die Umwelt zu verhindern.

Bei der Beurteilung der organischen Belastung von Wasser bzw. Abwasser werden üblicherweise Summenparameter (DOC, CSB, AOX) berücksichtigt, wobei hierbei eine Überwachung von Einzelsubstanzen nicht möglich ist. Ein solches Vorgehen erfordert eine aufwendige Analytik, die in mehreren Schritten durchgeführt werden muß. Nach der Probenahme können solche Verbindungen durch die verschiedenen bekannten chromatographischen Verfahren getrennt und mit verschiedenen Detektoren angezeigt werden.

Neben anderen bekannten Anreicherungsmethoden ist die sogenannte Festphasenmikroextraktion für solche Analyseverfahren bekannt. Mögliche Verfahren und Vorrichtungen für die Festphasenmikroextraktion sind beispielsweise in WO 91/15745, US 5,496,741 und US 5,576,217 beschrieben.

Dabei wird eine in einer spritzenähnlichen Vorrichtung aufgenommene Quarzfaser, die zumindestens teilweise mit einem Polymer beschichtet ist, verwendet, wobei im beschichteten Bereich die Festphasenmikroextraktion erfolgt.

Nach erfolgter Extraktion kann dann mit den bekannten gaschromatographischen Verfahren die Detektion verschiedener Inhaltsstoffe durchgeführt werden.

Bei den bisher bekannten Verfahren bzw. Vorgehensweisen werden jedoch nicht alle Einflußgrößen berücksichtigt. Außerdem ist ein für die verschiedensten Anwendungsfälle aber erforderlicher automatisierter Betrieb, der die Probenahme, Probenvorbereitung und Quantifizierung mit einschließt, bei einer entsprechenden Überwachung über längere Zeiträume mit geringem manuellen Überwachungsaufwand nicht möglich.

Aus diesem Grunde ist es Aufgabe der Erfindung, eine Möglichkeit zu schaffen, mit der verschiedene anorganische und organische Inhaltsstoffe in Fluiden quasikontinuierlich im automatischen Betrieb mit hoher Detektionsgenauigkeit überwacht werden können.

Erfindungsgemäß wird diese Aufgabe mit den Merkmalen des Anspruchs 1 für das erfindungsgemäße Verfahren und den Merkmalen des Anspruchs 8 für eine entsprechend ausgebildete Vorrichtung gelöst. Vorteilhafte Ausgestaltungsformen und Weiterbildungen der Erfindung ergeben sich bei Nutzung der in den untergeordneten Ansprüchen enthaltenen Merkmale.

Erfindungsgemäß wird dabei so vorgegangen, daß das jeweilige Fluid, in dem organische oder anorganische Inhaltsstoffe enthalten sein können, mit einem definierten Volumen oder einem definierten Volumenstrom, wobei letzteres insbesondere für gasförmige Fluide der Fall sein soll, unter Verwendung der bekannten Festphasenmikroextraktion mit anschließender chromatographischer Trennung und Detektion der jeweiligen Inhaltsstoffe durchgeführt wird. Dabei erfolgt eine Temperierung des jeweiligen Fluides auf eine konstante Temperatur, so daß die Extraktion und Detektion unter Ausschluß des Temperatureinflusses erfolgen kann.

Weitere Möglichkeiten für den Ausschluß anderer Einflußgrößen bestehen darin, insbesondere für flüssige Fluide, den pH-Wert auf einen für die Extraktion und Analyse günstigen definierten Wert einzustellen, zusätzlich oder alternativ dem Fluid mindestens ein Reagenz zuzufügen, mit der gezielt chemische Reaktionen, z.B. eine Derivatisierung, im Fluid ausgelöst werden können, so daß weitergehende Analysemöglichkeiten erreicht werden können. Dabei sollte für die Einstellung des pH-Wertes je nach Bedarf Säure oder Base oder die chemische Reaktionen auslösenden Reagenzien dosiert zugegeben werden, wobei das jeweils zugegebene Volumen bei der Endauswertung, sprich der Quantifizierung der in der jeweiligen Fluidprobe enthaltenen Inhaltsstoffe berücksichtigt werden sollte. Für die Zugabe der Säure, Base oder Reagenzien kann auf bekannte Dosiereinrichtungen, wie sie bei ebenfalls bekannten Titrationsverfahren üblicherweise verwendet werden, zurückgegriffen werden.

Besonders günstig ist es, wenn dem zu überwachenden Fluid ein weiteres Fluid, das eine oder mehrere Referenzverbindungen (interne Standards) enthält, zugegeben wird. Dabei sind die Zusammensetzung und auch die jeweilige Konzentration sowie das zugegebene Volumen oder der Volumenstrom bekannt. Parallel zur Quantifizierung unbekannter Inhaltsstoffe wird die bekannte Konzentration des internen Standards ständig überwacht. Vom Sollwert abweichende Meßergebnisse des internen Standards lassen auf Störungen im gesamten Analysensystems schließen. Dadurch wird es möglich, bei der Durchführung des erfindungsgemäßen Verfahrens eventuell auftretende Fehler sehr schnell zu erkennen, so daß entsprechend reagiert werden kann.

Bei der Auswertung der Meßergebnisse kann auf diese Art und Weise festgestellt werden, ob bestimmte Substanzen bzw. Komponenten gegebenenfalls quantitativ richtig erfaßt worden sind und bei auftretenden Abweichungen dann entsprechend auf vorab aufgetretene Fehler geschlossen werden kann. Solche Fehler können beispielsweise eine nicht mehr funktionsfähige Faser für die Festphasenmikroextraktion, fehlende Hilfsfluide für die Beeinflussung des pH-Wertes, fehlende Reagenzien oder Undichtigkeiten, beispielsweise am Gaschromatographen, sein. Es wird also bei der Auswertung der Meßergebnisse die Konzentration des internen Standards ständig überwacht, damit bei Abweichungen vom Sollwert um einen bestimmten, vorher festzulegenden Betrag entsprechende Fehlermeldungen erfolgen und notwendige Maßnahmen ergriffen werden können.

Bei der Auswertung, die günstigerweise in einer elektronischen Auswerte- und Steuereinheit, z.B. einem Computer erfolgt, kann ein entsprechendes Alarmsignal ausgelöst werden, so daß das Bedienpersonal in die Lage versetzt wird, den Fehler kurzfristig zu lokalisieren und entsprechend zu reagieren.

Mit der erwähnten zentralen Auswerte- und Steuereinheit kann so das Verfahren voll automatisiert über einen längeren Zeitraum betrieben werden, ohne daß eine ständige Überwachung erfolgen muß. Mit der elektronischen Auswerte- und Steuereinheit können die einzelnen Verfahrensschritte:
- Probenahme, Probenvorbereitung (z.B. Titration), Festphasenmikroextraktion, chromatische Trennung, Detektion und gegebenenfalls die Auslösung eines Alarmes bei Erfassung eines gefährlichen Stoffes oder beim Überschreiten von vorgebbaren Grenzwerten für bestimmte Stoffe
vollautomatisiert durchgeführt werden. Die elektronische Auswerte- und Steuereinheit steuert dabei das Volumen bzw. den Volumenstrom des zu überwachenden Fluides, die Dosierung der zuzugebenden Fluide bzw. Reagenzien, die Intervalle, in denen die Festphasenmikroextraktion und die chromatographische Trennung und Detektion durchgeführt werden. Außerdem kann in einer Gefahrensituation entweder, wie bereits erwähnt, Alarm ausgelöst oder, wie am Beispiel Abwasser beschrieben, der Zulauf in die Kläranlage oder der Ablauf in die Umwelt abgesperrt werden.

Mit zusätzlichen Dosiereinrichtungen können Hilfslösungen oder bekannte Standardlösungen eingesetzt werden, die insbesondere für alternierend durchzuführende Kalibrierungen verwendet werden können. Dabei kann anstelle des eigentlich zu untersuchenden bzw. zu überwachenden Fluides deionisiertes Wasser verwendet und mit einer solchen Standardlösung versetzt werden, die dann in der bekannten Zusammensetzung ohne weiteres geeignete Kalibrierwerte liefert, die dann für nachfolgende wieder mit Probenfluid durchgeführte Messungen herangezogen werden können (externe Kalibrierung).

Bekannte Mengen verschiedener Referenzbindungen können direkt in die Probe gegeben werden, um einerseits die Inhaltsstoffe zu quantifizieren (interne Kalibrierung) und andererseits die Leistungsfähigkeit des Gesamtsystems zu überwachen.

Außerdem ist es günstig, um ein beschleunigtes Erreichen des Absorptionsgleichgewichtes bei der Extraktion zu erreichen, flüssige Proben zu rühren oder Ultraschall auszusetzen, um die sich ansonsten um die für die Festphasenmikroextraktion verwendete Faser ausbildende Diffusionsgrenzschicht zu minimieren.

Nach der Extraktion können die verschiedensten bekannten chromatographischen Analyseverfahren wie z.B.

Gaschromatographie mit den verschiedensten bekannten Detektionsverfahren wie AED, ECD, FID, NPD, MS eingesetzt und die verschiedenen entsprechend getrennten Inhaltsstoffe einzeln erfaßt und überwacht werden. Für die Überwachung kann auf entsprechend bekannte elektronische Auswertungsverfahren zurückgegriffen werden, die es ermöglichen, eine Überwachung der verschiedensten Fluidformen automatisiert durchzuführen. Dabei können Probenahme, Meßanalytik örtlich getrennt von der Auswerteeinrichtung eingesetzt werden, da eine Meßwertübertragung mit den aus der Kommunikationstechnik bekannten Möglichkeiten ohne weiteres erfolgen kann. Wird von der Auswerteeinrichtung die Überschreitung von zumindest einem Grenzwert eines gefährlichen Inhaltsstoffes erfaßt, kann von dieser zumindest ein Alarm, günstigerweise jedoch ein Steuersignal ausgelöst werden, so daß auf Gefahrensituationen durch beispielsweise Absperren von Zu- oder Abflüssen rechtzeitig reagiert werden kann.

Wird das erfindungsgemäße Verfahren für die Überwachung von gasförmigen Fluiden eingesetzt, können diese, falls erforderlich, vor der Extraktion getrocknet, gegebenenfalls einer Derivatisierung unterzogen und temperiert werden. Dabei ist es für die Temperierung gasförmiger Fluide günstig, diese im Gegenstrom durch eine Heizschlange zu führen und durch entsprechenden Wärmeaustausch das gasförmige Fluid der für die Festphasenmikroextraktion verwendeten Faser mit definierter konstanter Temperatur zuzuführen.

Bei der Überwachung von flüssigen Fluiden werden erfindungsgemäß Gefäße verwendet, die mit einem Heizmantel umgeben sind, durch das ein Heizmittel (z.B. erwärmtes Wasser) zur Temperierung des temporär in den Gefäßen aufgenommenen flüssigen Fluides eingesetzt wird. Die Temperierung kann aber auch mit einem Kühlmittel durchgeführt werden.

Für die Extraktion kann die hierfür verwendete Faser sowohl in ein gasförmiges als auch ein flüssiges Fluid eintauchen, wie auch im Dampfraum oberhalb des Flüssigkeitsspiegels angeordnet werden, um gasförmige, gegebenenfalls verdampfte Komponenten zu extrahieren und zu detektieren.

Mit der bereits erwähnten Derivatisierung können Verbindungen in dem zu überwachenden Fluid in leichter zu extrahierende Verbindungen überführt werden und außerdem können ionisch vorliegende anorganische Verbindungen, wie z.B. Schwermetallionen, in neutrale, metallorganische Verbindungen überführt und dann mittels der Festphasenmikroextraktion extrahiert werden.

Das erfindungsgemäße Verfahren und mögliche dazu zu verwendende Vorrichtungen, die für Ausführungsbeispiele noch nachfolgend besser beschrieben werden, können für die quasi-kontinuierliche Analyse von Trinkwasser, Oberflächenwasser und Abwasser, in der Lebensmittelindustrie, beispielsweise dort im Getränkesektor automatisiert eingesetzt werden. Dabei bietet sich das erfindungsgemäße Verfahren für den letzten Anwendungsfall bei der Prozeßkontrolle an, wobei die Festphasenmikroextraktion hierbei bevorzugt im Dampfraum oberhalb des Flüssigkeitspegels durchgeführt werden sollte.

Das erfindungsgemäße Verfahren sollte vorteilhaft in zwei voneinder getrennten jedoch für das jeweilige Fluid miteinander verbundenen Gefäßen durchgeführt werden. Dabei soll die Vorbereitung der Probe in einem und die Extraktion in einem anderen Gefäß durchgeführt werden. Beide Gefäße können für die Einstellung einer probenspezifischen Temperatur temperierbar sein. Das Gefäß für die Extraktion kann eine einfach ausgebildete Füllstandregelung, mit der der Füllstand variabel eingestellt werden kann aufweisen. Außerdem ist es möglich, mit diesen beiden Gefäßen Fluide durch intermittierende Extraktion und chromatographische Auswertung über längere Zeiträume zu analysieren, ohne daß manuell eingegriffen werden muß. Es können Extraktionen sowohl direkt aus dem Fluid, wie auch im Dampfraum oberhalb des Fluides durch die bereits genannte Füllstandsvariationsmöglichkeit im Extraktionsgefäß durchgeführt werden. Wird der gewünschte Füllstand im Gefäß erreicht, kann umgehend entweder die entsprechende Pumpe mittels der Auswerte- und Steuereinheit abgeschaltet oder die Pumpe durch öffnen bzw. schließen von Ventilen, ebenfalls von der Auswerte- und Steuereinheit aktiviert, kurzgeschlossen werden, so daß keine weitere Zuführung von Fluid in das Gefäß danach erfolgt. Bei Verwendung einer Zweikanalschlauchpumpe oder zweier Pumpen mit gleicher Drehzahl und gleichen Schlauchinnendurchmesser ist ein Abschalten der Pumpen nicht notwendig, da in das Extraktionsgefäß pro Zeiteinheit genausoviel hinein wie herausgepumpt wird und damit das Volumen ebenfalls konstant bleibt.

Die Erfindung zeichnet sich durch große Wartungsintervalle aus. Es ist lediglich die Zugabe der Mittel für die Einstellung des pH-Wertes, der Hilfslösung und/oder der Reagenzien sowie von Zeit zu Zeit ein Austausch der Faser erforderlich. Ein unbeaufsichtigter Betrieb über eine Dauer von mindestens einer Woche ist möglich.

Es müssen keine toxischen oder anderweitig stark gefährdenden Chemikalien (z.B. toxische organische Lösungsmittel) verwendet werden. Neben den Betriebskosten für den Chromatographen sind nur noch relativ geringe Kosten für die verschiedenen zuzugegebenden Reagenzien zu berücksichtigen.

Nachfolgend soll die Erfindung an Ausführungsbeispielen näher beschrieben werden.

Dabei zeigen:
- Figur 1: den schematischen Aufbau eines Beispiels einer erfindungsgemäßen Vorrichtung, mit der organische oder anorganische Inhaltsstoffe in Abwasser detektiert werden können;
- Figur 2: ein für eine Festphasenmikroextraktion verwendbares Gefäß, das bei der Überwachung von flüssigen Proben einsetzbar ist und bei dem die für die Festphasenmikroextraktion verwendete Faser in die Flüssigkeit eintaucht und
- Figur 3: das in Figur 2 gezeigte Gefäß, wobei hier die für die Festphasenmikroextraktion verwendete Faser im Dampfraum oberhalb des Flüssigkeitspegels angeordnet ist.

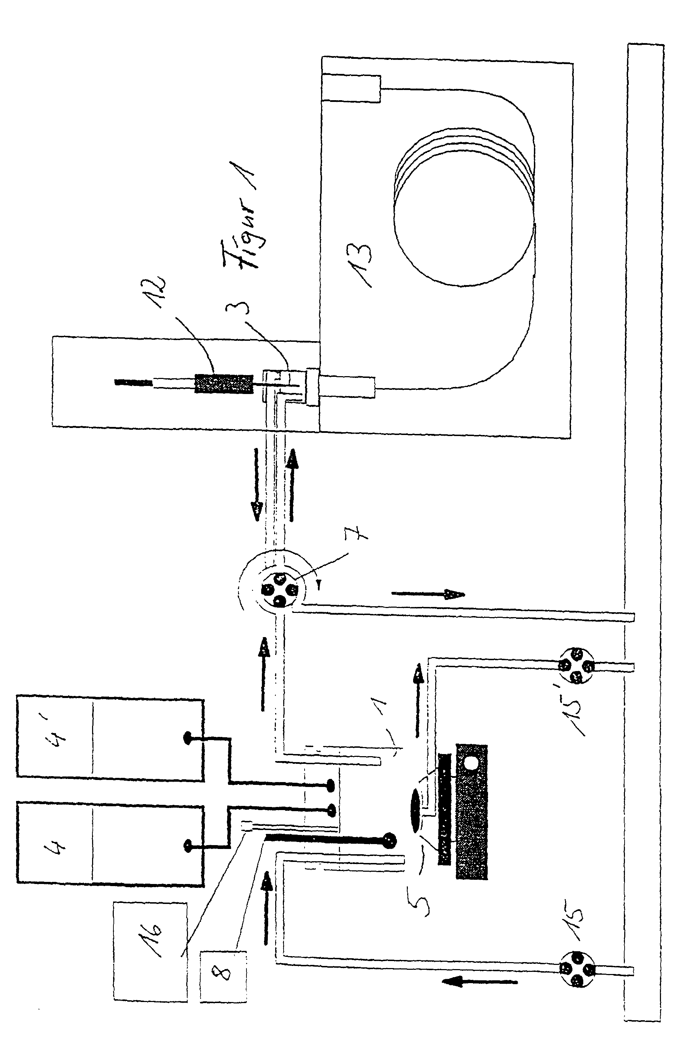

In der Figur 1 ist ein Beispiel einer Anwendung einer erfindungsgemäßen Vorrichtung in Verbindung mit dem erfindungsgemäßen Verfahren zur Überwachung von Abwasser, vor der Zuführung in eine Kläranlage dargestellt.

Dabei wird eine repräsentative Abwasserprobe aus einer Abwasserleitung 14 mittels einer Pumpe 15 in ein Gefäß 1 gefördert.

Im Gefäß 1 ist ein pH-Wert-Sensor 8 vorhanden, der über eine nicht dargestellte Steuerung eine Dosiereinrichtung 4 beeinflußt, in der bei diesem Beispiel Natronlauge enthalten ist und diese in gesteuerter Form eine Endpunkttitration auf den pH-Wert 10 durchführt, um z.B. Aminoverbindungen in die neutrale Form zu bringen. Dabei wird das hierfür erforderliche Volumen an Natronlauge bestimmt und bei der Endauswertung berücksichtigt.

Das mit dem entsprechenden pH-Wert von 10 eingestellte Abwasser gelangt aus dem Gefäß 1 unter Verwendung einer Zweikanalschlauchpumpe 7 zur Festphasenmikroextraktion, eine zumindest teilweise polymerbeschichtete Quarzfaser 3, die von einem Faserhalter 12 gehalten wird und die erforderliche Extraktion der Verbindungen des Abwassers wird durchgeführt, bis das Extraktionsgleichgewicht an der Faser 3 im mit Polymer beschichteten Bereich erreicht ist. Bei genügend hohen Konzentrationen kann die Extraktion jedoch bereits lange vor Erreichen des Extraktionsgleichgewichtes abgebrochen werden. Nach der Extraktion wird das Abwasser dann über den zweiten Kanal mittels der Zweikanalschlauchpumpe 7 wieder in den Abwasserkanal 14 zurückgeführt.

Mit der Dosiereinrichtung 4 oder einer zusätzlichen nicht dargestellten Dosiereinrichtung kann auch ein interner Standard oder mehrere interne Standards dem zu überwachenden Abwasser dosiert zugegeben werden, mit dem bei bekannter Zusammensetzung bzw. Kenntnis der Konzentration der darin enthaltenen Stoffe eine Überwachung des Gesamtverfahrens erfolgen kann. Abweichungen vom Sollwert werden automatisch erkannt, so daß die Fehlerursachen schnell behoben werden können.

Dabei wird der der Faser 3 zur Extraktion zugeführte Volumenstrom über die gezielte Beeinflussung der Drehzahl der Zweikanalpumpe 7 und durch die Verwendung von Verbindungsschläuchen mit kalibriertem Innendurchmesser auf einen definiert vorgebbaren Wert eingestellt und eingehalten.

Alternativ kann der Volumenstrom für die Dauer der Extraktion durch Stoppen der Pumpe angehalten werden.

Nach Einstellung des Extraktionsgleichgewichtes an der Faser 3 kann mit einem bekannten Gaschromatographen 13 die Detektion der einzelnen anorganischen bzw. organischen Inhaltsstoffe im Abwasser durchgeführt werden. Werden bei dieser Detektion Grenzwertüberschreitungen bestimmter gefährlicher Inhaltsstoffe festgestellt, kann über eine nicht dargestellte Steuer- und Auswerteeinheit der Abwasserkanal 14 vor der Kläranlage gesperrt oder umgeleitet werden, so daß eine Havarie oder Störung in der Kläranlage vermieden wird.

Im Gefäß 1 ist weiter eine Rühreinrichtung 5 enthalten, die aus einem außerhalb des Gefäßes 1 angeordneten Magnetrührer und einem im Gefäß 1 vorhandenen Magnetrührstab, der bevorzugt mit Glas oder PTFE beschichtet ist, besteht. Der Magnetrührer besteht z.B. aus einem Minielektromotor (z.B. 6 V Nennspannung), in dem ein Antriebsmagnet aufgesetzt ist und bei dessen Drehung der sich im Gefäß 1 befindende Magnetrührer in starke Rotation versetzt. Dabei kann vorteilhaft die Drehzahl des Magnetrührstabes im Gefäß 1 gesteuert und/oder geregelt werden. Das vom Magnetrührer im Gefäß eingenommene Volumen kann bei der Auswertung berücksichtigt werden.

In der Figur 1 ist außerdem erkennbar, daß die Probenflüssigkeit, in diesem Fall das Abwasser, aus dem Gefäß 1 mit einer weiteren Schlauchpumpe 15' wieder entfernt werden kann.

Mit dieser Lösung ist es auch möglich, nach Entleerung des Gefäßes 1, mit einem oder mehreren Spülvorgängen deionisiertes Wasser zuzuführen und aus der zweiten in der Figur 1 dargestellten Dosiereinrichtung 4' eine in ihrer Konsistenz bekannte Standardlösung definierten Volumens zuzugeben, so daß diese Lösung wieder über die Schlauchpumpe 7 der Festphasenmikroextraktion und der chromatographischen Auswertung zugeführt und mit den so erhaltenen Meßwerten eine Kalibrierung durchgeführt werden kann.

Anstelle oder zusätzlich zur Dosiereinrichtung 4' kann eine andere Dosiereinrichtung, in der ein ganz bestimmtes Reagenz aufgenommen ist, am Gefäß 1 vorhanden sein. Mit dieser Dosiereinrichtung kann wiederum eine Reagenz in das Gefäß 1 in das zu überwachende Abwasser dosiert werden und zumindest eine chemische Reaktion ausgelöst werden. Dabei kann es sich bevorzugt um eine Derivatisierung handeln, um bessere Voraussetzung für die Erfassung bestimmter Inhaltsstoffe zu erreichen.

Desweiteren ist in der Figur 1 die Anordnung eines Niveauschalters 16 im Gefäß 1 dargestellt, mit dem eine Füllstandsregelung bzw. Füllstandsbegrenzung im Gefäß 1 möglich ist.

Durch einen Ablauf, der über eine Leitung mit der Schlauchpumpe 15' verbunden ist, wird, wie bereits erwähnt, das Abwasser aus dem Gefäß 1 abgeführt und es können außerdem nach der Titration zur Einstellung des pH-Wertes in den alkalischen Bereich eventuell ausfallende Hydroxidniederschläge abgeführt werden.

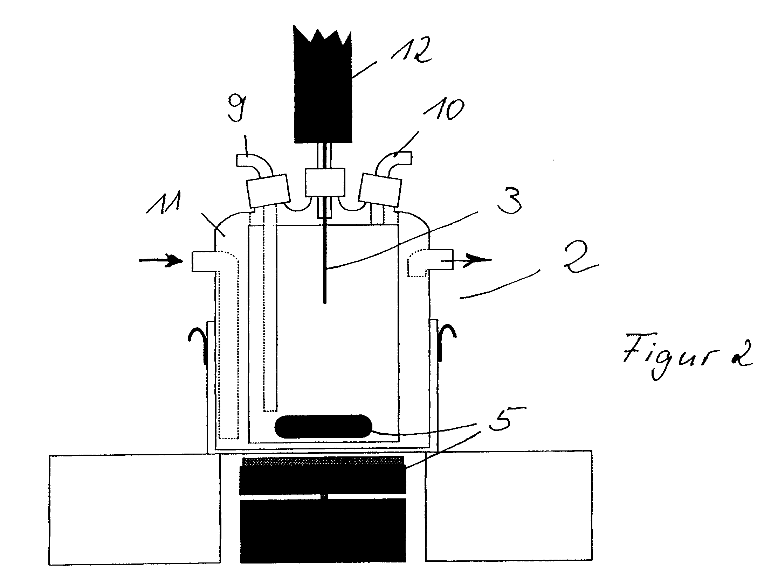

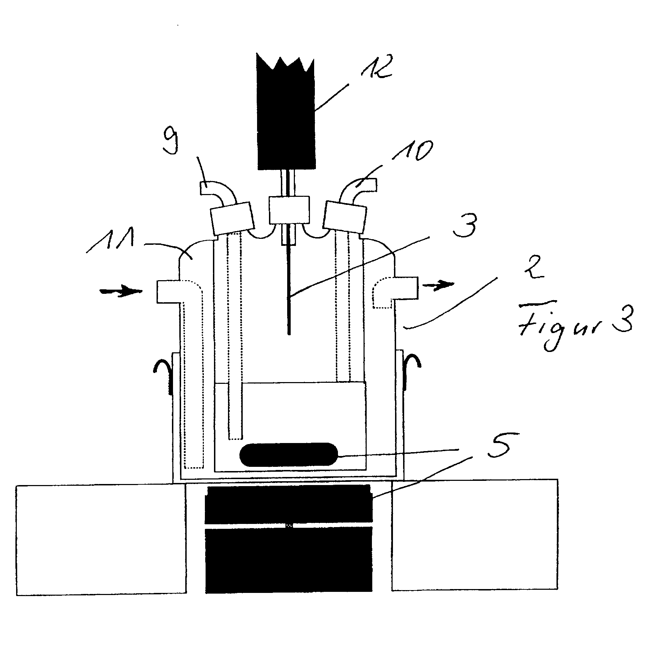

In den Figuren 2 und 3 ist ein zweites Gefäß 2 dargestellt, in dem die Faser 3 einmal in eine Flüssigkeit eingetaucht (Figur 2) und einmal im Dampfraum oberhalb des Flüssigkeitspegels (Figur 3) angeordnet ist.

In ein solches Gefäß 2 kann die entsprechend vorbereitete Probe über einen Probeneinlauf 9 ein- und über einen Probenablauf 10 abgeführt werden. Hierbei kann es sich um nach außen abgedichtete Rohre oder Schläuche handeln, deren Aus- bzw. Eintrittsöffnungen in unterschiedlicher Höhe angeordnet sind, wobei dadurch eine einfache Füllstandsregelung im Zusammenwirken mit einer hier nicht dargestellten Pumpe erreicht werden kann. Der Füllstand kann durch einfache Variation der Höhe der Öffnung des Rohres für den Probenauslauf 10 variert werden. Zum Befüllen des Gefäßes 2 in dem die Extraktion durchgeführt wird, wird mit einer Zweikanalpumpe und mit Schläuchen deren Innendurchmesser kalibriert ist, gearbeitet. Dadurch können die Füll- und Absaugrate gleich groß gehalten werden.

Während der Befüllung wird so lange Luft abgesaugt, bis die Flüssigkeit die Eintrittsöffnung des Rohres für den Probenauslauf 10 erreicht hat. Daraus folgt, daß nach Füllung des Gefäßes ein gleich großer Volumenstrom in das Gefäß 2 gefördert, wie abgeführt wird. Der Pegel im Gefäß 2 bleibt konstant.

Über die Länge bzw. die Eintauchtiefe des Rohres für den Probenauslauf 10 kann die Füllhöhe eingestellt werden.

Nach dem Befüllen kann die Pumpe abgeschaltet oder kurzgeschlossen werden und die Extraktion kann beginnen. Nach der Extraktion durch die Faser 3 kann die chromatographische Trennung und Detektion einzelner Inhaltsstoffe durchgeführt werden.

Dabei ist es günstig, die Flüssigkeit im Gefäß 2 vor der Extraktion auf eine bestimmte Temperatur zu temperieren. Hierfür ist das Gefäß 2 mit einem Heizmantel 11 umgeben, durch den ein Heizmittel, z.B. erwärmtes Wasser geführt werden kann und die Temperierung der Flüssigkeit im Gefäß 2 unter Verwendung eines nicht dargestellten Thermostaten mit einer Regelung für die Heizmittelzufuhr durchgeführt werden kann. Eine entsprechende Temperierung kann auch bei dem in Figur 1 gezeigten Gefäß 1 eingesetzt werden.

Das Gefäß 2 und die anderen um, an oder in diesem angeordneten Einzelelemente sind günstigerweise aus Glas in Verbindung mit Silikon- oder PTFE-Dichtelementen hergestellt. Das Gefäß 2 hat beispielsweise ein Volumen im Inneren von ca. 5 bis 10 mL, wobei das Füllvolumen etwas kleiner ist und für die Extraktion im Dampfraum oberhalb des Pegels gemäß Figur 3 variabel einstellbar ist.

Auch am und im Gefäß 2 ist eine Magnetrühreinrichtung 5 vorhanden, die entsprechend der bei der Beschreibung der Figur 1 Beschriebenen ausgebildet ist und in erster Linie zur Reduzierung der Diffusionsgrenzschicht um die Faser 3 dient, so daß die für die Extraktion erforderliche Zeit verkürzt werden kann.

Die Faser 3 ist in an sich bekannter Anordnung von einem Faserhalter 12 im Gefäß 2 gehalten. Die Faser 3 kann automatisch intermittierend aus dem Gefäß 2 entfernt und der chromatographischen Untersuchung zugeführt werden. Im Anschluß daran kann sie wieder für einen neuen Meßlauf verwendet werden, wobei die Anzahl der möglichen Wiederverwendungen im dreistelligen Bereich liegt.

## Patentansprüche

1. Verfahren zur quasi-kontinuierlichen Bestimmung von anorganischen oder organischen Inhaltsstoffen in Fluiden, bei dem das Fluid, dem ein weiteres Fluid, das als interne(n) Standard(s) eine oder mehrere Referenzverbindung(en) in bekannter Konzentration enthält, mit vorgebbarem Volumen oder Volumenstrom zugegeben wird, mit definiertem Volumen oder Volumenstrom zugeführt und dann eine Festphasenmikroextraktion mit anschließender chromatographischer Trennung und Detektion der Inhaltsstoffe intermittierend durchgeführt wird,
**dadurch gekennzeichnet , daß** das Fluid auf eine vorgebbare Temperatur temperiert, durch dosierte Zugabe von Säuren oder Basen ein definierter pH-Wert eingestellt und/oder zumindest ein eine chemische Reaktion im Fluid auslösendes Reagenz dosiert zugegeben wird und daß flüssige Proben während der Festphasenmikroextraktion zur Einstellung des Extraktionsgleichgewichtes gerührt und/oder Ultraschall ausgesetzt werden.

2. Verfahren nach Anspruch 1,
**dadurch gekennzeichnet, daß** Fluidzuführung, Dosierung, Temperierung, Festphasenmikroextraktion, Chromatographische Trennung und Detektion und die Auswertung mittels einer Auswerte- und Steuereinheit automatisiert durchgeführt werden.

3. Verfahren nach einem der Ansprüche 1 oder 2,
**dadurch gekennzeichnet, daß** alternierend Referenzmessungen zur Kalibrierung durchgeführt werden.

4. Verfahren nach Anspruch 1 bis 3,
**dadurch gekennzeichnet, daß** die quantitative Detektion der Inhaltsstoffe durch Vergleich mit empirisch ermittelten Vergleichswerten durchgeführt wird.

5. Verfahren nach einem der Ansprüche 1 bis 4,
**dadurch gekennzeichnet, daß** bei der Auswertung das zugegebene Volumen der Säure, Base, Reagenzie und/oder einer Hilfslösung in bezug zum Probenvolumen oder Volumenstrom berücksichtigt wird.

6. Verfahren nach einem der Ansprüche 1 bis 5,
**dadurch gekennzeichnet, daß** gasförmige Fluide vor der Festphasenmikroextraktion getrocknet werden.

7. Verfahren nach einem der Ansprüche 1 bis 6,
**dadurch gekennzeichnet, daß** die Festphasenmikroextraktion durch Eintauchen einer polymerbeschichteten Faser (3) in das Fluid oder Eintauchen in einen Dampfraum oberhalb eines flüssigen Fluids durchgeführt wird.

8. Vorrichtung zur Durchführung des Verfahrens nach einem der Ansprüche 1 bis 7,
**dadurch gekennzeichnet,**
**daß** in einem ersten Gefäß (1) eine Rühreinrichtung oder Ultraschallsonde und ein ph-Wert-Sensor (8) angeordnet sind, und daß das Gefäß (1) mit einer Dosiereinrichtung (4) zur Zugabe eines Fluides, das einen oder mehrere interne Standards enthält, vorhanden ist.

9. Vorrichtung nach Anspruch 8,
**dadurch gekennzeichnet, daß** die Faser (3) in einem zweiten Gefäß (2) angeordnet ist.

10. Vorrichtung nach Anspruch 8 oder 9,
**dadurch gekennzeichnet, daß** am/im ersten Gefäß (1) mindestens eine weitere Dosierainrichtung (4') angeordnet ist.

11. Vorrichtung nach einem der Ansprüche 8 bis 10,
**dadurch gekennzeichnet, daß** die Rühreinrichtung (5) oder Ultraschallsonde im ersten und/oder zweiten Gefäß (1, 2) angeordnet ist/sind.

12. Vorrichtung nach Anspruch 10 oder 11,
**dadurch gekennzeichnet, daß** im ersten Gefäß (1) und/oder zweiten Gefäß (2) eine Füllstandsregelung vorhanden ist/sind.

13. Vorrichtung nach Anspruch 12,
**dadurch gekennzeichnet, daß** ein Fluidzulauf (9) und ein Fluidauslauf (10), deren Öffnungen in unterschiedlicher Höhe angeordnet werden können, im zweiten Gefäß (2) vorhanden sind.

14. Vorrichtung nach Anspruch 13,
**dadurch gekennzeichnet, daß** die Höhe der Öffnung des Probenauslaufs (10) im Gefäß (2) variabel einstellbar ist.

15. Vorrichtung nach einem der Ansprüche 8 bis 14,
**dadurch gekennzeichnet, daß** das Fluid über Schläuche mit kalibriertem Innendurchmeser mittels mindestens einer steuerbaren Pumpe (7) zur Faser (3) förderbar ist.

16. Vorrichtung nach einem der Ansprüche 8 bis 15,
**dadurch gekennzeichnet, daß** das erste und/oder zweite Gefäß (1, 2) mit einem durch eine die jeweilige Gefäßwand umgebenden Heizmantel (11) führbaren Kühl-/Heizmittel temperierbar ist/sind.

17. Vorrichtung nach einem der Ansprüche 8 bis 16,
**dadurch gekennzeichnet, daß** Gase mittels einer Heizschlange temperierbar sind.

## Claims

1. Method for quasi-continuous analysis of inorganic or organic substances contained in fluids, where the fluid, to which is added in a given volume or volume flow an additional fluid which contains as internal standard(s) one or more reference compounds in a known concentration, is delivered with defined volume or volume flow, and then a solid phase micro extraction with subsequent chromatographic separation and analysis of the content substances is intermittently carried out, **characterised in that** the fluid is tempered to a given temperature, a defined pH value is set by apportioned addition of acids or lye and/or a reagent which triggers a chemical reaction in the fluid is apportioned and the liquid samples are during the solid phase micro extraction agitated and/or subjected to ultrasound in order to establish the extraction balance.

2. Method according to Claim 1, **characterised in that** fluid delivery, apportioning, tempering, solid phase micro extraction, chromatographic separation and analysis and evaluation are carried out automatically by means of an evaluation and control unit.

3. Method according to one of Claims 1 or 2, **characterised in that** reference measurings are alternately carried out for calibration purposes.

4. Method according to Claim 1 to 3, **characterised in that** the quantitative analysis of the content substances is carried out by way of comparison with empirically determined comparative values.

5. Method according to one of Claims 1 to 4, **characterised in that** during the evaluation the added volume in acid, lye, reagent and/or an auxiliary solution are taken into consideration relative to the sample volume or volume flow.

6. Method according to one of Claims 1 to 5, **characterised in that** the gaseous fluids are dried prior to solid phase micro extraction.

7. Method according to one of Claims 1 to 6, **characterised in that** the solid phase micro extraction is carried out but immersing a polymer coated fibre (4) into the fluid or immersing into a vapour chamber above a liquid fluid.

8. Device for carrying out the method according to one of Claims 1 to 7, **characterised in that** a first vessel (1) contains an agitating device or an ultrasound probe and a ph-value sensor (8), and the vessel (1) is provided with an apportioning device (4) for adding a fluid which contains one or more internal standards.

9. Device according to Claim 8, **characterised in that** the fibre (3) is accommodated in a second vessel (2).

10. Device according to Claim 8 or 9, **characterised in that** at/in the first vessel (1) is accommodated at least one additional apportioning device (4').

11. Device according to one of Claims 8 to 10, **characterised in that** the agitating device (5) or ultrasound probe is/are accommodated in the first and/or second vessel (1, 2).

12. Device according to Claim 10 or 11, **characterised in that** in the first vessel (1) and/or in the second vessel (2) is accommodated a filling level control.

13. Device according to Claim 12, **characterised in that** a fluid delivery (9) and a fluid discharge (10), the openings of which can be arranged at different heights, are in the second vessel (2).

14. Device according to Claim 13, **characterised in that** the height of the opening of the sample discharge (10) in the vessel (2) can be variably set.

15. Device according to one of Claims 8 to 14, **characterised in that** the fluid is conveyed via hoses with calibrated inside diameter by means of at least one controllable pump (7) to the fibre (3).

16. Device according to one of Claims 8 to 15, **characterised in that** the first and/or second vessel (1, 2) can be tempered by means of a cooling/heating media ducted through a tempering sleeve (11) which encases a respective vessel wall.

17. Device according to one of Claims 8 to 16, **characterised in that** the gas is tempered by means of a tempering coil.

## Revendications

1. Procédé pour déterminer, de manière quasi continue, des substances organiques ou inorganiques contenues dans des fluides, dans lequel le fluide est acheminé avec volume ou courant volumique pouvant être prescrit auquel est ajouté, avec volume ou courant volumique pouvant être prescrit, un autre fluide qui contient, dans une concentration connue, un ou plusieurs composés de référence servant de standard(s) interne(s), et dans lequel une micro-extraction en phase solide suivie d'une séparation chromatographique et d'une détection des substances contenues sont effectuées par intermittence,
**caractérisé en ce que** le fluide est tempéré à une température qui peut être présélectionnée, est réglé sur un pH défini par addition dosée d'acides ou de bases et/ou au moins un réactif, déclenchant une réaction chimique dans le fluide, est ajouté de manière dosée et **en ce que** des échantillons liquides sont remués et/ou soumis à ultrasons pendant la micro-extraction en phase solide, pour régler l'équilibre d'extraction.

2. Procédé selon la revendication 1, **caractérisé en ce que** l'alimentation du fluide, le dosage, la mise à température, la micro-extraction en phase solide, la séparation chromatographique et la détection ainsi que l'évaluation sont effectués de manière automatisée au moyen d'une unité d' évaluation et de commande.

3. Procédé selon l'une des revendications 1 ou 2, **caractérisé en ce que** des mesures de référence sont effectuées en alternance en vue du calibrage.

4. Procédé selon la revendication 1 à 3, **caractérisé en ce que** la détection quantitative des substances contenues s'effectue par comparaison avec des valeurs de référence déterminées de manière empirique.

5. Procédé selon l'une des revendications 1 à 4, **caractérisé en ce que** pendant l'interprétation le volume ajouté d'acides, de bases, de réactifs et/ou d'une solution auxiliaire, par rapport au volume des échantillons ou le courant volumique, est pris en compte.

6. Procédé selon l'une des revendications 1 à 5, **caractérisé en ce que** les fluides sous forme gazeuse sont séchés avant la micro-extraction en phase solide.

7. Procédé selon l'une des revendications 1 à 6, **caractérisé en ce que** la micro-extraction en phase solide s'effectue par immersion d'une fibre (3) à revêtement de polymère dans le fluide, ou immersion dans un espace à vapeur au-dessus d'un fluide liquide.

8. Dispositif pour la mise en oeuvre du procédé selon l'une des revendications 1 à 7, **caractérisé en ce que** dans un premier récipient (1) sont disposés un dispositif d'agitation ou une sonde à ultrasons et un capteur de pH (8), et **en ce que** le récipient (1) comporte un dispositif de dosage (4) pour l'ajout d'un fluide qui contient un ou plusieurs standards internes.

9. Dispositif selon la revendication 8, **caractérisé en ce que** la fibre (3) est disposée dans un deuxième récipient (2).

10. Dispositif selon la revendication 8 ou 9, **caractérisé en ce que** sur/dans le premier récipient (1) est disposé au moins un autre dispositif de dosage (4').

11. Dispositif selon l'une des revendications 8 à 10, **caractérisé en ce que** le dispositif d'agitation (5) ou la sonde à ultrasons est disposé dans le premier et/ou le deuxième récipient (1, 2).

12. Dispositif selon la revendication 10 ou 11, **caractérisé en ce que** dans le premier récipient (1) et/ou le deuxième récipient (2), est prévue une régulation du niveau de remplissage.

13. Dispositif selon la revendication 12, **caractérisé en ce que** dans le deuxième récipient (2) sont prévus une arrivée de fluide (9) et une sortie de fluide (10) dont les ouvertures peuvent être disposées à des hauteurs différentes.

14. Dispositif selon la revendication 13, **caractérisé en ce que** la hauteur de l'ouverture de la sortie (10) des échantillons peut être réglée dans le récipient (2) de manière variable.

15. Dispositif selon l'une des revendications 8 à 14, **caractérisé en ce que** le fluide peut être transporté vers la fibre (3), par des tuyaux au diamètre intérieur calibré, au moyen d'au moins une pompe (7) qui peut être commandée.

16. Dispositif selon l'une des revendications 8 à 15, **caractérisé en ce que** le premier et/ou le deuxième récipient (1,2) peut/peuvent être tempéré(s) au moyen d'un fluide de refroidissement/chauffage qui peut être conduit à travers une enveloppe chauffante (11) entourant la paroi respective du récipient.

17. Dispositif selon l'une des revendications 8 à 16, **caractérisé en ce que** des gaz peuvent être tempérés au moyen d'un serpentin de chauffage.
